# EUROPEAN PATENT SPECIFICATION

(11) **EP 1 746 114 B1**
(45) Date of publication and mention of the grant of the patent: **13.06.2012**
(21) Application number: 05734728.8
(22) Date of filing: 21.04.2005
(51) Int. Cl.: C08F 220/58, C08F 236/14, C09K 3/00

(54) **WATER-SOLUBLE THICKENER AND COSMETIC PREPARATION CONTAINING SAME**
WASSERLÖSLICHER VERDICKER UND DIESEN ENTHALTENDE KOSMETISCHE ZUBEREITUNG
EPAISSISSEUR SOLUBLE DANS L'EAU ET PREPARATION COSMETIQUE CONTENANT LEDIT PRODUIT

(30) Priority: 10.05.2004 JP 2004139555
(43) Date of publication of application: 24.01.2007
(73) Proprietor: SHISEIDO COMPANY, LTD., Tokyo 104-8010 (JP)
(72) Inventor: SOGABE, Atsushi Shiseido Res. Cntr (Shin-Yokohama), Yokohama-shi Kanagawa 2248558 (JP); KANEDA, Isamu Shiseido Res. Cntr (Shin-Yokohama), Yokohama-shi Kanagawa 2248558 (JP)
(74) Representative: Merkle, Gebhard
(86) International application number: PCT/JP2005/007603
(87) International publication number: WO 2005/108521

(56) References cited:
- JP-A- 1 310 087
- JP-A- 10 279 636
- JP-A- 10 279 636
- JP-A- 57 171 494
- JP-A- 2001 115 135
- DATABASE WPI Week 200142 Thomson Scientific, London, GB; AN 2001-393062 XP002566698 & JP 2001 115135 A (SHISEIDO CO LTD) 24 April 2001 (2001-04-24)

## Description

### TECHNICAL FIELD

The present invention relates to water-soluble thickeners. More specifically, it relates to water-soluble thickeners that exhibit excellent usability in that they do not produce a sticky feel when in cosmetic preparations over a wide pH range. The present invention also relates to cosmetics with excellent usability, in which the water-soluble thickener has been blended.

### BACKGROUND ART

Examples of water-soluble thickeners that can be used in general fields such as pharmaceuticals and cosmetics include natural polymers such as various types of polysaccharides and gelatin, synthetic polymers such as polyoxyethylene and crosslinked poly(meth)acrylic acid, and inorganic ore such as montmorillonite and silica.

Of these, crosslinked poly(meth)acrylic acid in particular is inexpensive, has a good thickening effect, and gelatinizes at small amounts, and thus it is frequently used in the pharmaceutical and cosmetic industries, and particularly in cosmetics, as a water-soluble thickener or stabilizer.

However, dissociation of the carboxyl group of the crosslinked poly(meth)acrylic acid is suppressed in aqueous solutions that are acidic with a pH of 5 or less or that include salts, and this severely lowers its viscosity and it loses the ability to gel. It therefore cannot be used in preparations that require acidic conditions or that include salts.

In particular, this characteristic may be a fatal flaw for thickeners that are used for cosmetics, for which usability is important. For example, to retain the thickening effect in acidic conditions of pH 5 or less, or in the presence of salts, the amount that is blended must but significantly increased, and this has a noticeable adverse effect on the usability of the cosmetic. In other words, thickeners produce a sticky feel when they are applied to skin, and stickiness is a very serious problem in terms of the usability of a cosmetic.

To solve this problem, a copolymer of acrylamidealkylsulfonic acid and (meth)acrylic acid (Patent Document 1), a copolymer of acrylamidealkylsulfonic acid and monomers containing alkyl groups (Patent Document 2), and a homopolymer of 2-acrylamido-2-methylpropanesulfonic acid (Patent Document 3), among others, have been used in cosmetics.

However, although the polymer having the acrylamidealkylsulfonic acid backbone has improved acid resistance and can be used in preparations that require acidic conditions, it produces a sticky feel when dry, probably due to the acrylic acid, and thus cannot be said to have a sufficiently satisfactory usability as a thickener for cosmetics.

Patent Document 1: JP H9-157130 A
Patent Document 2: JP H10-279636 A
Patent Document 3: JP H10-67640 A

JP 2001 115135 discloses a water-soluble thickener that consists of a copolymer obtained by copolymerizing 2-acrylamide-2-methyl propane sulfonic acid or its salt, hydroxyethyl methacrylate and a crosslinkable monomer.

JP 10 279636 discloses thickener based on acrylic_terpolymer, obtained by copolymerisation of a sulfonic acid based monomer, a long-chain acrylate ester and N-hydroxyethylacrylamide, for use in cosmetics or skin care products, said copolymer is less sensitive to variation of pH and the cosmetic does not cause unpleasant stickiness.

### DISCLOSURE OF INVENTION

### [Problem that the present invention aims to solve]

In light of the foregoing matters, the inventors performed keen investigations to find a substance that can be used in cosmetics that require acidic conditions or the presence of a salt, and that in these exhibits a good thickening effect and has excellent usability, and through their efforts found that these conditions could be met by blending a copolymer that is obtained by copolymerizing 2-acrylamido-2-methylpropanesulfonic acid or its salt, hydroxyethylacrylamide, and a crosslinking monomer, into a cosmetic as a water-soluble thickener, and moreover, found that the copolymer that is obtained is a non-toxic, safe water-soluble thickener, and from these findings the inventors arrived at the present invention.

It is an object of the invention to provide a copolymer serving as a water-soluble thickener that has good stability and is safe and that can exhibit sufficiently satisfactory usability when a copolymer that has an acrylamidealkylsulfonic acid unit as its backbone is blended into a cosmetic as a water-soluble thickener.

### [Means to solve the Problem]

In other words, the invention provides a water-soluble thickener made of a copolymer that is obtained by copolymerizing 2-acrylamido-2-methylpropanesulfonic acid or its salt, hydroxyethylacrylamide, and a crosslinking monomer, or is made of a copolymer that is obtained by further neutralizing said copolymer that has been obtained using an alkaline agent.

The invention also provides the foregoing water-soluble thickener, in which the crosslinking monomer is N,N'-methylenebisacrylamide.

The invention also provides the foregoing water-soluble thickener, in which a mole ratio of the 2-acrylamido-2-methylpropanesulfonic acid unit and the hydroxyethylacrylamide unit in the copolymer is 1:9 to 9:1.

Further, the invention also provides a cosmetic in which the foregoing water-soluble thickener has been blended.

### [Effects of the invention]

With the water-soluble thickener of the invention, it is possible to stably thicken cosmetic preparations over a wide pH range without lowering the viscosity, and it is possible to provide an excellent usage feel when applied that cannot be obtained when conventional thickeners are blended.

### BEST MODE FOR CARRYING OUT THE INVENTION

The present invention is described in detail below.

In the invention, the copolymer that is used as a water-soluble thickener is a cross-linked binary copolymer that is obtained by copolymerizing 2-acrylamido-2-methylpropanesulfonic acid or its salt, hydroxyethylacrylamide, and a crosslinking monomer.

A salt of 2-acrylamido-2-methylpropanesulfonic acid may be used, and that salt can be used alone or together with 2-acrylamido-2-methylpropanesulfonic acid in the copolymerization with hydroxyethylacrylamide. As the salt of 2-acrylamido-2-methylpropanesulfonic acid, it is possible to use alkali metals, ammonia, and organic amine salts such as triethylamine or triethanolamine, for example.

It should be noted that in this invention, the 2-acrylamido-2-methylpropanesulfonic acid unit of the copolymer that is obtained can be neutralized with an alkaline agent and adopted as the water-soluble thickener.

The hydroxyethylacrylamide used in the invention is expressed by the following chemical formula.

**CH₂=CH-CO-NH-CH₂CH₂-OH** [Chemical formula 1]

The crosslinking monomer used in the invention includes at least two polymerizable double bonds in the molecule, and it is essential that the crosslinking monomer efficiently produces a crosslinked structure in the polymer system between 2-acrylamido-2-methylpropanesulfonic acid or its salt and hydroxyethylacrylamide.

Examples of the crosslinking monomer include ethylene glycol diacrylate, ethylene glycol dimethacrylate, polyoxyethylene diacrylate, polyoxyethylene dimethacrylate, diethylene glycol dimethacrylate, trimethylolpropane triacrylate, N,N'-methylenebisacrylamide, N,N'-ethylenebisacrylamide, triallyl isocyanurate, and pentaerythrithol dimethacrylate, and it is possible to use one, two, or more selected from among these.
In the present invention, the use of N,N'-methylenebisacrylamide is particularly preferable.

The mole ratio of the 2-acrylamido-2-methylpropanesulfonic acid unit and the hydroxyethylacrylamide unit in the copolymer, that is the water-soluble thickener of the invention, preferably is 1:9 to 9:1. The viscosity of the water-soluble thickener of the invention is the result of extension of the molecule chains due to electrostatic repulsion by the sulfonyl group, which is a strongly dissociating group, and the crosslinked structure that results from the crosslinked monomers, and if the content of the 2-acrylamido-2-methylpropanesulfonic acid unit or its salt is less than 10 mol% compared to the hydroxyethylacrylamide unit, then the molecule chains are not sufficiently extend and as a result it is no longer possible to obtain sufficient viscosity.

The amount of crosslinking monomer that is added is preferably in the range of 0.0001 to 2.0 mol% with respect to the total number of moles of the 2-acrylamido-2-methylpropanesulfonic acid or its salt and hydroxyethylacrylamide. If it is less than 0.0001 mol%, then the ability of the water-soluble thickener that has been prepared to gelatinize may be low. If the water-soluble thickener that is produced includes the crosslinking monomer at more than 2.0 mol% and is dispersed in water, then it gelatinizes elastically and thus it may not be possible to obtain the desired usability.

As for the polymerization method for the copolymerization, it is possible to carry out the polymerization using a method known to the public such as solution polymerization, suspension polymerization, bulk polymerization, or emulsion polymerization. There are no particular limitations regarding the polymerization initiator as long as it has the ability to initiate radical polymerization, and possible examples thereof include benzoyl peroxide, azobisisobutyronitrile, potassium persulfate, and ammonium persulfate.

The copolymer can have been obtained through a normal homogenous polymerization system, or it can have been obtained through a heterogeneous polymerization system known as inverse phase emulsion polymerization.

However, in this invention, the copolymer that is obtained by inverse phase emulsion polymerization is obtained as a polymer microgel, and thus it is preferable that this microgel is used in applications as the water-soluble thickener.

That is to say, the water-soluble thickener that is obtained by inverse phase emulsion polymerization is a thickener made of a synthetic polymer microgel that is obtained by dissolving the monomers in the dispersion phase of a composition in which an organic solvent or an oil serves as the dispersion solvent and water serves as the dispersion phase, and then the radical polymerization was occurred in the dispersion phase.

The microgel is fine particles of the synthetic polymer electrolyte made of the copolymer, and swells in water, ethanol, or water/ethanol mixture solutions to produce a highly viscous solution that appears homogenous to the naked eye. Further, it is different from the copolymer that is obtained by a homogeneous polymerization system in that it does not have to be crushed into a powder when it is blended into a cosmetic as a water-soluble thickener, and in that it exhibits an enhanced thickening effect and produces an excellent feel when used. It is also preferable in terms of the appearance of the cosmetic. In particular, it exhibits an excellent thickening effect even in cosmetics that include a high amount of ethanol. Blending the microgel with liniment compositions for skin coloring that include dihydroxyacetone (transparent gels, emulsified gels, creams, emulsion-type self-tanning cosmetics) makes it possible to provide stable compositions that exhibit a very good thickening effect and coloring effect, and thus is preferable.

In the heterogeneous polymerization system known as inverse phase emulsion polymerization, it is preferable to produce the water-soluble thickener consisting of the microgel by using a surfactant that has been suitably adjusted to a selected hydrophilicity/lipophilicity balance (HLB) so that the inverse emulsion polymerization system forms a single phase microemulsion or a fine W/O emulsion.

A single phase microemulsion is a state in which the oil phase and the water phase coexist in a thermodynamically stable manner and the surface tension between the oil and water is at a minimum. A fine W/0 emulsion is a state in which the oil and the water are present as a fine W/0 emulsion in a thermodynamically unstable but kinetically stable manner. In general, the particle diameter of the inner water phase of a fine W/0 emulsion is about several tens to 100 nm. These states are determined solely by the system composition and the temperature, and are not affected by the mechanical agitation conditions, for example.

The composition that makes up the heterogeneous polymerization system is made of a dispersion solvent (which constitutes the outer phase) that is made from an organic solvent or an oil component that does not mix with water, and a dispersion phase (which constitutes the inner phase) that is made of water.
Examples of preferable organic solvents include alkanes such as pentane, hexane, heptane, octane, nonane, decane, and undecane; cycloalkanes such as cyclopentane, cyclohexane, cycloheptane, and cyclooctane; and aromatic and cyclic hydrocarbons such as benzene, toluene, xylene, decaline, and naphthalene.
Examples of preferable oil components include non-polar oil components such as paraffin oil.

The monomers are dissolved in water, that is, the dispersion phase, to produce a monomer aqueous solution, and then this is mixed with the organic solvent or the oil component serving as the dispersion solvent and heated to a desired temperature, after which the polymerization initiator is added to the water phase to carry out the polymerization.

In general, with heterogeneous polymerization methods, it is known that the physical properties of the polymer that is manufactured are different depending on the agitation conditions during the polymerization. This is because the emulsion system is not thermodynamically stable and as a result the shape and the size of the emulsified particles change depending on the agitation conditions. In the present invention, it was found that these problems can be avoided by carrying out the polymerization in the thermodynamically stable single phase microemulsion region or the metastable fine W/0 emulsion region near the single phase region. Specifically, it has become possible to obtain a microgel with a good thickening effect by polymerizing the polymer in a fine water phase (water droplets) by adjusting the composition of the polymerization system (organic solvent type, HLB of the surfactant) in such a manner that the single phase microemulsion region or the fine W/0 emulsion region appears near the optimum polymerization temperature for the polymerization initiator for an ordinary thermal polymerization or redox polymerization.

The molecular weight of the water-soluble thickener that is obtained is 100,000 or more, and is adjusted according to the crosslinking agent that is added and the desired viscosity.

The cosmetic of the invention is manufactured by mixing the water-soluble thickener into a formulation. The amount of water-soluble thickener that is blended may be suitably determined in accordance with the target cosmetic, but from the standpoint of usability, the preferable blend amount is 0.01 to 10 wt%, and more preferably 0.1 to 5 wt%.

The cosmetic of the present invention can be prepared using a common method in accordance with the target agent shape by suitably blending the water-soluble thickener, as necessary, with other components that are normally used in cosmetics, such as powder components, liquid fats and oils, solid fats and oils, waxes, hydrocarbons, higher fatty acids, higher alcohols, ester oils, silicone oils, anionic surfactants, cationic surfactants, amphoteric surfactants, nonionic surfactants, humectants, water-soluble polymers, thickeners, coating agents, ultraviolet light absorbents, sequestering agents, lower alcohols, polyhydric alcohols, sugars, amino acids, organic amines, polymer emulsions, pH adjusting agents, skin nutrients, vitamins, antioxidants, antioxidation assistants, perfumes, and water.
Specific examples of the components with which the water-soluble thickener may be blended are listed below, and it is possible to prepare the endermic liniment of the invention by blending the above essential components and any one or more of the following components.

Examples of powder components include inorganic powders (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstic acid metal salt, magnesium, silica, zeolite, barium sulfate, firing calcium sulfate (calcined gypsum), calcium phosphate, fluorine-apatite, hydroxy apatite, ceramic powder, metallic soaps (such as myristic acid zinc, calcium palmitate, and aluminum stearate), and boron nitride); organic powders (such as polyamide resin powder (nylon powder), polyethylene powder, poly methyl methacrylate powder, polystyrene powder, powders of copolymer resin of styrene and acrylic acid, benzoguanamine resin powder, polytetrafluoroethylene powder, and cellulose powder); inorganic white pigments (such as titanium dioxide and zinc oxide); inorganic red pigments such as iron oxide (red iron oxide) and iron titanate); inorganic brown pigments (such as γ-iron oxide); inorganic yellow pigments (such as yellow iron oxide and loess); inorganic black pigments (such as black iron oxide and low oxides of titanium); inorganic purple pigments (such as manganese violet and cobalt violet); inorganic green pigments (such as chromium oxide, chromium hydroxide, and cobalt titanate); inorganic blue pigments (such as ultramarine blue and indigo blue); pearl pigment (such as titanium oxide coated mica, titanium oxide coated bismuth oxychloride, titanium oxide coated talc, colored titanium oxide coated mica, bismuth oxychloride, fish scale flakes); metal powder pigments (such as aluminum powder and copper powder); organic pigments such as zirconium, barium or aluminum rake (for example, organic pigments such as red 201, red 202, red 204, red 205, red 220, red 226, red 228, red 405, orange 203, orange 204, yellow 205, yellow 401 and blue 404, as well as red 3, red 104, red 106, red 227, red 230, red 401, red 505, orange 205, yellow 4, yellow 5, yellow 202, yellow 203, green 3 and blue 1); and natural colors (such as chlorophyll and β -carotene).

Examples of the liquid fats and oils include avocado oil, tsubaki oil, turtle fatty acid, macademia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil, rice bran oil, Chinese tung oil, Japanese tung oil, jojoba oil, germ oil, and triglycerin.

Examples of the solid fats and oils include cacao butter, coconut oil, hydrogenated coconut oil, palm oil, palm kernel oil, Japanese core wax nucleus oil, hydrogenated oil, Japanese core wax, and hydrogenated castor oil.

Examples of waxes include beeswax, candelilla wax, cotton wax, carnauba wax, bayberry wax, insect wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin ethyl alcohol ether, ceresin, and microcrystalline wax.

Examples of hydrocarbon oils include liquid paraffin, ozocerite, squalane, pristane, paraffin, ceresin, squalene, and petrolatum.

Examples of the higher fatty acids include lauric acid, myristic acid, palmitic acid, stearic acid, behenic acid, oleic acid, undecylenic acid, isostearic acid, linolic acid, linoleic acid, eicosapentaenoic acid (EPA), and docosahexaenoic acid (DHA).

Examples of higher alcohols include straight-chain alcohols (such as lauryl alcohol, cetyl alcohol, stearyl alcohol, behenyl alcohol, myristyl alcohol, oleyl alcohol, and cetostearyl alcohol) and branched-chain alcohols (such as mono stearyl glycerin ether (batyl alcohol), 2-decyltetradecynol, lanolin alcohol, cholesterol, phytosterol, hexyl dodecanol, isostearyl alcohol, and octyl dodecanol).

Examples of ester oils include isopropyl myristate, cetyl octanoate, octyl dodecyl myristate, isopropyl palmitate, butyl stearate, hexyl laurate, myristil myristate, decyl oleate, dimethyl hexyl decyl octanoate, cetyl lactate, myristil lactate, lanolin acetate, isocetyl stearate, isocetyl isostearate, cholesteryl hydroxy 12-stearate, di-2-ethylene glycol ethylhexanoate, dipentaerythritol fatty acid ester, N-alkylene glycol monoisostearate, neopentyl glycol dicaprate, diisostearyl malate, glycerin di-2-heptylundecanoate, trimethylolpropane tri-2-ethylhexanoate, trimethylolpropane triisostearate, tetra-2-pentaerythritol ethylhexanoate, glycerin tri-2-ethylhexanoate, glycerin trioctanoate, glycerin triisopalmitate, trimethylolpropane triisostearate, cetyl 2-ethyl hexanoate, 2-ethylhexyl palmitate, glycerin trimyristate, tri-2-heptyl undecanoate glyceride, methyl castor oil fatty acid, oleyl oleate, aceto glyceride, 2-heptyl undecyl palmitate, diisobutyl adipate, 2-octyldodecyl N-lauroyl-L-glutamate, di-2-heptyl undecyl adipate, ethyl laurate, di-2-ethylhexyl sebacate, 2-hexyl decyl myristate, 2-hexyl decyl palmitate, 2-hexyl decyl adipate, diisopropyl sebacate, 2-ethylhexyl succinate, and triethyl citrate.

Examples of silicone oils include chain polysiloxanes (for example, dimethylpolysiloxane, methylphenyl polysiloxane, and diphenyl polysiloxane); cyclic polysiloxanes (for example, octamethylcyclotetrasiloxane, decamethylcyclopentasiloxane, and dodecamethylcyclohexasiloxane), silicone resins forming a three-dimensional network, silicone rubber, and various modified polysiloxanes (amino-modified polysiloxane, polyether-modified polysiloxane, alkyl-modified polysiloxane, and fluorine-modified polysiloxane).

Examples of anionic surfactants include fatty acid soaps (for example, sodium laurate and sodium palmitate); higher alkyl sulfuric ester salts (for example, sodium lauryl sulfate and potassium laurylsulfate); alkylether sulfuric ester salts (for example, POE-triethanolamine laurylsulfate, sodium POE-lauryl sulfate); N-acyl sarcosinic acids (for example, sodium lauroyl sarcosinate); higher fatty acid amidosulfonic acid salts (for example, sodium N-myristoyl-N-methyl taurate, sodium palm oil fatty acid methyl taurate, and sodium lauryl methyl taurate); phosphate ester salts (sodium POE-oleyl ether phosphate, POE-stearyl ether phosphoric acid, etc.); sulfosuccinates (for example, sodium di-2-ethylhexylsulfosuccinate, sodium mono lauroyl mono ethanol amide polyoxyethylene sulfosuccinate, and sodium lauryl polypropylene glycol sulfosuccinate); alkylbenzene sulfonates (for example, sodium linear dodecylbenzenesulfonate, triethanolamine linear dodecylbenzenesulfonate, and linear dodecylbenzenesulfonic acid); higher fatty acid ester sulfates (for example, hydrogenated palm oil fatty acid glycerine sodium sulfate): N-acyl glutamates (for example, mono sodium N-lauroylglutamate, disodium N-stearoylglutamate, and mono sodium N-myristoyl-L-glutamate) ; sulfated oils (for example, turkey red oil); POE-alkylether carboxylic acid; POE-alkylarylether carboxylate; α-olefin sulfonate; higher fatty acid ester sulfonates; sec-alcohol sulfates; higher fatty acid alkylol amide sulfate esters; sodium lauroyl monoethanolamine succinates; and ditriethanolamine N-palmitoylaspartate; and sodium caseinate.

Examples of cationic surfactants include alkyltrimethylammonium salts (for example, stearyltrimethyl ammonium chloride and lauryltrimethyl ammonium chloride); alkylpyridinium salts (for example, cetylpyridinium chloride); distearyldimethylammonium dialkyldimethylammonium chloride; poly (N,N'-dimethyl-3,5-methylene piperidinium) chloride; alkyl quaternary ammonium salts; alkyl dimethylbenzyl ammonium salts; alkyl isoquinolinium salts; dialkylmorpholine salts; POE-alkylamines; alkylamine salts; polyamine fatty acid derivatives; amylalcohol fatty acid derivatives; benzalkonium chloride; and benzethonium chloride.

Examples of amphoteric surfactants include imidazoline-type ampholytic surfactants (such as 2-undecyl-N,N,N-(hydroxyethyl carboxymethyl)-2-imidazoline sodium salt and 2-cocoyl-2-imidazolinium hydroxide-1-carboxyethyloxy 2 sodium salt), and betaine-type surfactants (such as 2-heptadecyl-N-carboxymethyl-N-hydroxyethyl imidazolinium betaine, lauryldimethylamino betaine acetate, alkyl betaine, amide betaine, and sulfobetaine).

Examples of lipophilic nonionic surfactants include sorbitan fatty acid esters (for example, sorbitan mono oleate, sorbitan mono isostearate, sorbitan mono laurate, sorbitan mono palmitate, sorbitan mono stearate, sorbitan sesqui oleate, sorbitan trioleate, diglycerol sorbitan penta-2-ethylhexylate, diglycerol sorbitan tetra-2-ethylhexylate) ; glycerin polyglycerin aliphatic acids (for example, mono cottonseed oil fatty acid glycerine, glyceryl monoerucate, glycerin sesquioleate, glyceryl monostearate, α, α' - glycerin oleate pyroglutamate, monostearate glycerine malic acid); propylene glycol fatty acid esters (for example, propylene glycol monostearate); hydrogenated castor oil derivatives; and glycerin alkylethers.

Examples of hydrophilic nonionic surfactants include POE-sorbitan fatty acid esters (for example, POE-sorbitan monooleate, POE-sorbitan monostearate, POE-sorbitan monoolate, and POE-sorbitan tetraoleate); POE sorbitol fatty acid esters (for example, POE sorbitol monolaurate, POE-sorbitol monooleate, POE-sorbitolpentaoleate, and POE-sorbitol monostearate); POE-glycerin fatty acid esters (for example, POE-monooleates such as POE-glycerin monostearate, POE-glycerin monoisostearate, and POE-glycerin triisostearate); POE-fatty acid esters (for example, POE-distearate, POE-monodioleate, and ethylene glycol distearate); POE-alkylethers (for example, POE-lauryl ether, POE-oleyl ether, POE-stearyl ether, POE-behenyl ether, POE-2-octyl dodecyl ether, and POE-cholestanol ether); pluaronics (for example, pluaronic); POE·POP-alkylethers (for example, POE·POP-cetyl ether, POE·POP-2-decyl tetradecyl ether, POE·POP-monobutyl ether, POE·POP-lanolin hydrate, and POE·POP-glycerin ether); tetra POE·tetra POP-ethylenediamino condensates (for example, tetronic); POE-castor oil hydrogenated castor oil derivatives (for example, POE-castor oil, POE-hydrogenated castor oil, POE-hydrogenated castor oil monoisostearate, POE-hydrogenated castor oil triisostearate, POE-hydrogenated castor oil monopyroglutamic monoisostearic diester, and POE-hydrogenated castor oil maleic acid); POE-beeswax·lanolin derivatives (for example, POE-sorbitol beeswax); alkanol amides (for example, palm oil fatty acid diethanol amide, laurate monoethanolamide, and fatty acid isopropanol amide); POE-propylene glycol fatty acid esters; POE-alkylamines; POE-fatty acid amides; sucrose fatty acid esters; alkyl ethoxydimethylamine oxides; and trioleyl phosphoric acid.

Examples of humectants include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl-12-hydroxy stearate, sodium lactate, bile salt, d1-pyrrolidone carboxylic acid salt, short chain soluble collagen, diglycerin (EO)PO adduct, chestnut rose extract, yarrow extract, and sweet clover extract.

Examples of natural water-soluble polymers include plant-based polymers (such as gum arabic, gum tragacanth, galactan, guar gum, carob gum, karaya gum, carrageenan, pectin, agar, quince seed (Cydonia oblonga), algae colloids (brown algae extract), starches (rice, corn, potato, and wheat), and glycyrrhizic acid); microorganism-based polymers (for example, xanthan gum, dextran, succinoglucan, and pullulan); and other polymers (for example, fish collagen, fish gelatin, wheat protein, and silk protein).

Examples of semi synthetic water-soluble polymers include starch-type polymers (for example, carboxymethyl starch and methylhydroxypropyl starch); cellulose-type polymers (for example, methyl cellulose, ethyl cellulose, methylhydroxypropyl cellulose, hydroxyethyl cellulose, cellulose sodium sulfate, hydroxypropyl cellulose, carboxymethyl-cellulose, sodium carboxymethyl cellulose, crystal cellulose, and cellulose powder); and alginic acid-type polymers (for example, sodium alginate and propyleneglycol alginate).

Examples of synthetic water-soluble polymers include vinyl polymers (for example, polyvinyl alcohol, polyvinyl methyl ether, polyvinylpyrrolidone, and carboxy vinyl polymer); polyoxyethylene polymers (for example, a copolymer of polyethylene glycol 20,000, 40,000, or 60,000 and polyoxyethylene polyoxypropylene); acrylic polymers (for example, sodium polyacrylate, polyethylacrylate, and polyacrylamide); polyethyleneimine ; and cationic polymers.

Examples of thickeners include gum arabic, carrageenan, karaya gum, gum tragacanth, carob gum, quince seed (Cydonia oblonga), casein, dextrin, gelatin, sodium pectate, sodium alginate, methyl cellulose, ethyl cellulose, CMC, hydroxy ethyl cellulose, hydroxypropyl cellulose, PVA, PVM, PVP, sodium polyacrylate, carboxy vinyl polymer, locust bean gum, guar gum, tamarind gum, cellulose dialkyl dimethylammonium sulfate, xanthan gum, aluminum magnesium silicate, bentonite, hectorite, AlMg silicate (beagum), laponite, and silicic acid anhydride.
It should be noted that in the present invention, it is only necessary to blend the water-soluble thickener of the invention, and thus it is not absolutely necessary that these thickeners are added. However, it is possible to add these to the cosmetic of the invention.

Examples of the ultraviolet absorbents include the following compounds.

### (1) Benzoic acid ultraviolet light absorbents

For example, paraminobenzoic acid (hereafter abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester.

### (2) Anthranilic acid ultraviolet light absorbents

For example, homo mentyl-N-acetyl anthranilate.

### (3) Salicylic acid ultraviolet light absorbents

For example, amyl salicylate, mentyl salicylate, homo mentyl salicylate, octyl salicylate, phenyl salicylate, benzyl salicylate, and *p*-isopropanol phenyl salicylate).

### (4) Cinnamic acid ultraviolet light absorbents

For example, octylcinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-*p*-methoxycinnamate (2-ethylhexyl-*p*-methoxycinnamate), 2-ethoxyethyl-p-methoxycinnamate, cyclohexyl-p-methoxycinnamate, ethyl-α-cyano-β -phenylcinnamate, 2-ethylhexyl-α-cyano- β-phenylcinnamate, and glyceryl mono-2-ethyl hexanoyl-diparamethoxycinnamate).

### (5) Triazine ultraviolet light absorbents

For example, bisresorcinyl triazine.

More specifically, bis{[4-(2-ethylhexyloxy)-2-hydroxy]phenyl}-6-(4-methoxyphenyl)-1,3,5-triazine, and 2, 4, 6-Tris {4-(2-ethylhexyloxycarbonyl)-anilino}-1, 3, 5-triazine.

### (6) Other ultraviolet light absorbents

For example, 3-(4'-methylbenzylidene)-d,1-camphor, 3-benzylidene-d,1-camphor, 2-phenyl-5-methyl benzoxazol, 2,2'-hydroxy-5-methylphenyl benzotriazol, 2-(2'-hydroxy-5'-t-octylphenyl) benzotriazol; 2-(2'-hydroxy-5'-methylphenyl benzotriazol), dianisoylmethane, 4-methoxy-4'-t-butyl dibenzoyl-methane, and 5-(3,3-dimethyl-2-norbornylidene)-3-pentane-2-one. Also, pyridazine derivatives such as dimorpholinopyridazinone.

Examples of sequestering agents include 1-hydroxy ethane-1,1-diphosphonic acid, 1-hydroxy ethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium ethylenediaminehydroxyethyl triacetate.

Examples of the lower alcohols include ethanol, propanol, isopropanol, isobutyl alcohol, and t-butyl alcohol.

Examples of polyhydric alcohols include dihydric alcohols (for example, ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (for example, glycerin and trimethylolpropane); tetrahydric alcohols (for example, pentaerythritols such as 1,2,6-hexanetriol); pentahydric alcohols (for example, xylitol); hexahydric alcohols (for example, sorbitol and mannitol); polyhydric alcohol polymers (for example, diethylene glycol, dipropylene glycol, triethylene glycol, polypropylene glycol, tetraethylene glycol, diglycerin, polyethylene glycol, triglycerin, tetraglycerin, and polyglycerin); dihydric alcohol alkylethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono 2-methyl hexyl ether, ethylene glycol isoamyl ether, ethylene glycol benzyl ether, ethylene glycol isopropyl ether, ethylene glycol dimethylether, ethylene glycol diethyl ether, and ethylene glycol dibutyl ether); dihydric alcohol alkylethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerin mono alkyl ethers (for example, chimyl alcohol, selachyl alcohol, and batyl alcohol); sugar alcohols (for example, sorbitol, maltitol, maltotriose, mannitol, sucrose, erythritol, glucose, fructose, starch amylolysis sugar, maltose, xylitose, and starch amylolysis sugar reduction alcohols); glysolid; tetrahydro furfuryl alcohol; POE-tetrahydro furfuryl alcohol; POP-butyl ether; POP·POE-butyl ether; tripoli oxypropylene glycerin ether; POP-glycerin ether; POP-glycerin ether phosphoric acid; POP·POE-pentane erythritol ether, and polyglycerin.

Examples of monosaccharides include trioses (for example, D-glyceryl aldehyde and dihydroxyacetone); tetroses (for example, D-erythrose, D-erythrulose, D-threose, and erythritol); pentoses (for example, L-arabinose, D-xylose, L-lyxose, D-arabinose, D-ribose, D-ribulose, D-xylulose, and L-xylulose); hexoses (for example, D-glucose, D-talose, D-psicose, D-galactose, D-fructose, L-galactose, L-mannose, and D-tagatose); heptoses (for example, aldoheptose and heprose); octoses (for example, octurose); deoxysugars (for example, 2-deoxy-D-ribose, 6-deoxy-L-galactose, and 6-deoxy-L-mannose); amino sugars (for example, D-glucosamine, D-galactosamine, sialic acid, amino uronic acid, and muramic acid); and uronic acids (for example, D-glucuronic acid, D-mannuronic acid, L-guluronic acid, D-galacturonic acid, and L-iduronic acid).

Examples of oligosaccharides include sucrose, umbelliferose, lactose, planteose, isolignoses, α, α -trehalose, raffinose, lignoses, and stachyose verbascoses.

Examples of polysaccharides include cellulose, quince seed, chondroitin sulfate, starch, galactan, dermatan sulfate, glycogen, gum arabic, heparan sulfate, hyaluronic acid, traganth gum, keratan sulfate, chondroitin, xanthan gum, mucoitin sulfate, guar gum, dextran, kerato sulfate, locust bean gum, succinoglucan, and charonic acid.

Examples of amino acids include neutral amino acids (for example, threonine and cysteine) and basic amino acids (for example, hydroxylysine). Examples of amino acid derivatives include sodium acyl sarcosinate (sodium lauroyl sarcosinate), acyl glutamate, acyl β-sodium alanine, glutathione, and pyrrolidone carboxylic acid.

Examples of organic amines include monoethanolamine, diethanolamine, triethanolamine, morpholine, triisopropanolamine, 2-amino-2-carbinyl-1,3-propanediol, and 2-amino-2-methyl-1-propanol.

Examples of polymer emulsions include acrylic resin emulsions, ethyl polyacrylate emulsions, acryl resin liquids, polyacrylic alkyl ester emulsions, polyvinyl acetate resin emulsions, and natural rubber latex.

Examples of pH adjustment agents include buffers such as lactic acid-sodium lactate, citric acid-sodium citrate, and succinic acid-sodium succinate.

Examples of vitamins include vitamin A, B1, B2, B6, C and E as well as their derivatives, pantothenic acid and its derivatives, and biotin.

Examples of the antioxidants include tocopherols, dibutyl hydroxytoluene, butyl hydroxyanisole, and gallic ester.

Examples of antioxidation assistants include phosphoric acid, citric acid, ascorbic acid, maleic acid, malonic acid, succinic acid, fumaric acid, cephalin, hexametaphosphate, phytic acid, and ethylene diamine tetraacetic acid.

Examples of other components that may be blended include antiseptics (methylparaben, ethylparaben, butylparaben, and phenoxyparaben); anti-inflammatory agents (for example, glycyrrhizic acid derivatives, glycyrrhetinic acid derivatives, salicylic acid derivatives, hinokitiol, zinc oxide, and allantoin); whitening agents (for example, creeping saxifrage extract, arbutin, tranexamic acid, L-ascorbic acid, L-ascorbic acid phosphate magnesium salt, glucoside L-ascorbate, and potassium 4-methoxysalicylicate); various extracts (for example, phellodendron bark, goldthread, lithospermum root, Paeonia lactiflora, Swertia japonica, Birch, sage, loquat, carrot, aloe, Malva sylvestris, Iris, grape, coix ma-yuen, sponge gourd, lily, saffron, Cnidium officinale, sheng jiang, Hypericum erectum, Ononis, garlic, Guinea pepper, citrus unshiu peel, Ligusticum acutilobum, and seaweed), activators (royal jelly, photosensitive substances, and cholesterol derivatives); circulation promoting agents (for example, nicotinic acid benzyl esters, nicotinic acid β-butoxy ethyl esters, capsaicin, zingerone, cantharis tincture, ichthammol, tannic acid, α-borneol, tocopherol nicotinate, inositol hexanicotinate, cyclandelate, cinnarizine, tolazoline, acetylcholine, verapamil, cepharanthine, and γ-orizanol); anti-seborrhea agents (for example, sulfur and thiantol); anti-inflammatory agents (for example, tranexamic acid, thiotaurine, and hypotaurine); and bactericides (such as benzoic acid and its salts, isopropylmethylphenol, undecylenic acid and its salts, monoethanolamide undecylenate, cetyltrimethylammonium chloride, cetylpyridinium chloride, benzalkonium chloride, benzethonium chloride, alkyldiaminoethylglycine chloride, chlorhexidine chloride, orthophenylphenol, chlorhexidine gluconate, cresol, chloramine T, chlorxylenol, chlorcresol, chlorphenesin, chlorobutanol, 5-chloro-2-methyl-isothiazoline-3-one, salicylic acid and its salts, 1,3-dimethylol-5,5-dimethylhydantoin, alkylisoquinolinium bromide, domiphen bromide, sorbic acid and its salts, thymol, thiram, dehydroacetic acid and its salts, triclosan, trichlorocarbanilide, *p*-oxybenzoates, *p*-chlorophenol, halocarban, pyrogallol, phenol, hexachlorophene, 2-methyl-4-isothiazoline-3-one, NN"-methylenebis(N'-(3-hydroxymethyl-2, 5-dioxo-4-imidazolidinyl urea, sodium lauroylsarcosine, and resorcine).

The form that the cosmetic of the invention takes is not fixed, and as long as the above water-soluble thickener retains its function, any form may be adopted, including a solution, a soluble form, an emulsion, a power dispersion, a water-oil two layer form, and a water-oil-powder three layer form. The manner in which the product is embodied also is not restricted. In addition to base cosmetics, the scope of the invention also includes hair cosmetics and makeup cosmetics. By dissolving the water-soluble thickener in water and using this as a hydrophilic base, preferably it is possible to adjust skin lotion, cosmetic waters, and dyeing agents, for example. Further, by mixing this with an oil base and agitating the product, it is possible to adjust emulsion cosmetics.

### EXAMPLES

The present invention is described below in specific detail through examples, but the present invention is not limited by these examples. The blend ratios of the cosmetic preparations are expressed as a wt% of the overall weight.

### Example 1: Manufacturing the Water-Soluble Thickener

12.9 g of 2-acrylamido-2-methylpropanesulfonic acid (corresponds to 50 mol%) and 7.1 g of hydroxyethylacrylamide (corresponds to 50 mol%) were dissolved in 200 g pure water in a 500 ml separable flask, and in this was dissolved 0.019 g N,N'-methylenebisacrylamide (corresponds to 0.1 mol%). The reaction solution was refluxed in nitrogen gas for approximately 1 hour, 0.02 g potassium persulfate was dissolved in this, and then the temperature was raised to 60°C and the polymerization reaction was carried out for six hours. After the polymerization reaction was over, the solution was cooled to room temperature and then the paste-like polymer that was obtained was dried under reduced pressure and then pulverized to obtain the desired copolymer, which is the water-soluble thickener. It should be noted that this copolymer is a water-soluble thickener that is obtained through a homogenous polymerization system.

### Example 2: Manufacturing the Water-Soluble Thickener

In a 500-ml beaker, 35 g of hydroxyethylacrylamide (made by Kojin), 17.5 g of 2-acrylamido-2-methylpropanesulfonic acid (made by Sigma), and the methylenebisacrylamide were dissolved in 260 g ion-exchanged water and the pH was adjusted to 7.0 using sodium hydroxide, thereby preparing an aqueous monomer solution. 260 g of n-hexane, 8.7 g of polyoxyethylene (3) oleyl ether (EMALEX 503, made by Nihon Emulsion), and 17.6 g of polyoxyethylene (6) oleyl ether (EMALEX 506, made by Nihon Emulsion) were put into a 1,000 ml three-neck flask provided with a refluxing apparatus, these were mixed and dissolved, and then subjected to N₂ substitution. The aqueous monomer solution was added to this three-neck flask, and then the temperature was raised to 65 to 70°C with an oil bath while stirring in an N₂ atmosphere. When the system temperature had reached 65 to 70°C, it was confirmed that the system had become a semitransparent microemulsion, and then 0.1 g ammonium persulfate was added to the polymerization system to start the polymerization. The temperature of the polymerization system was kept at 65 to 70°C for three hours while stirring, producing the microgel. After the polymerization was finished, acetone was added to the microgel suspension to precipitate the microgel, and this was followed by rinsing with acetone three times to remove any remaining monomers and surfactant. The precipitate was filtered and then dried under reduced pressure to obtain a copolymer, that is, the water-soluble thickener, in the form of a white powder. This copolymer is a microgel that was obtained through a heterogeneous polymerization system using inverse phase emulsion polymerization.

### Comparative Example 1

Sodium polyacrylate (Hibis Wako 105, made by Wako Pure Chemical Industries) was used as the water-soluble thickener of Comparative Example 1.

### Comparative Example 2

The copolymer produced in Example 2 of the aforementioned Patent Document 1 (JP H9-157130 A) (which is a copolymer of 2-acrylamido-2-methylpropanesulfonic acid and acrylic acid that has been crosslinked by N,N'-methylenebisacrylamide) was used as the water-soluble thickener of Comparative Example 2.

### Test Example 1: Thickening Effects (pH stability)

The pH of a 0.5 wt% aqueous solution of the water-soluble thickeners of the examples and the comparative examples was adjusted with 10 N sodium hydroxide, and the viscosity of the reagent solution at the various pH levels was measured using a B-type viscometer (12 rpm, 1 min, 25°C) and these were compared. The results are shown in Table 1. From the results of Table 1 it can be understood that the water-soluble thickener of the present invention that was produced in Examples 1 and 2 retained a stable viscosity over all pH regions.

**[Table 1]**

| 0.5 wt% Aqueous Solution Viscosity (mPas) | | | | | | |
|---|---|---|---|---|---|---|
| pH | 2 | 3 | 5 | 7 | 8 | 10 |
| Comparative Example 1 | 160 | 1200 | 8000 | 11000 | 15000 | 14000 |
| Comparative Example 2 | 7800 | 7800 | 8000 | 8500 | 8500 | 8400 |
| Example 1 | 4400 | 4300 | 4400 | 4600 | 4600 | 4400 |
| Example 2 | 18000 | 19500 | 21000 | 22000 | 22000 | 23000 |

Next, cosmetics in which the water-soluble thickeners of the examples and the comparative examples are blended were produced and their stability and usability were assessed based on the following criteria. In the stability test, the state after storing the cosmetics for one month at 50°C was evaluated visually. The usability was evaluated through a sensory test performed by a panel of nine specialists.

### <Stability>

○: No change in external appearance whatsoever.
Δ A slight change in the external appearance was observed.
×: There was a change in the external appearance and a drop in the viscosity was observed.

### <Usability>

ⓞ : All nine panelists responded that there was no sticky feel and that the usability was excellent.
○ : Six to eight panelists responded that there was no sticky feel and that the usability was excellent.
Δ : Three to five panelists responded that there was no sticky feel and that the usability was excellent.
× : Two or fewer panelists responded that there was no sticky feel and that the usability was excellent.

### Whitening Skin Lotion (Examples 3 and 4, Comparative Examples 3 and 4)

The whitening skin lotions shown in Table 2 were produced by an ordinary method. The lotion components and the results of evaluating the lotions are shown. From the results of Table 2 it can be understood that the cosmetics of the invention have very excellent stability and usability.

**[Table 2]**

| | Example 3 | Example 4 | Comp. Ex. 3 | Comp. Ex. 4 |
|---|---|---|---|---|
| Liquid paraffin | 5 | 5 | 5 | 5 |
| Dimethylpolysiloxane | 3 | 3 | 3 | 3 |
| Squalane | 2 | 2 | 2 | 2 |
| Alcohol | 3 | 3 | 3 | 3 |
| Glycerin | 8 | 8 | 8 | 8 |
| Hydrogenated Castor Oil | 2 | 2 | 2 | 2 |
| Potassium hydroxide | 0.4 | 0.4 | 0.4 | 0.4 |
| Citric acid | 0.01 | 0.01 | 0.01 | 0.01 |
| Sodium citrate | 0.09 | 0.09 | 0.09 | 0.09 |
| Glucoside ascorbate | 2 | 2 | 2 | 2 |
| Methylparaben | 0.3 | 0.3 | 0.3 | 0.3 |
| Example 1 | 0.5 | | | |
| Example 2 | | 0.5 | | |
| Comparative Example 1 | | | 0.5 | |
| Comparative Example 2 | | | | 0.5 |
| Ion-exchange water | to 100 | to 100 | to 100 | to 100 |
| Stability | ○ | ○ | × | ○ |
| Usability | ○ | ○ | × | Δ |

### Whitening Gel (Examples 5 and 6, Comparative Examples 5 and 6)

The whitening gels shown in Table 3 were produced by an ordinary method. The gel components and the results of evaluating the gel are shown. From the results of Table 3 it can be understood that the cosmetics of the invention have very excellent stability and usability.

**[Table 3]**

| | Example 5 | Example 6 | Comp. Ex. 5 | Comp. Ex. 6 |
|---|---|---|---|---|
| Dipropyleneglycol | 7 | 7 | 7 | 7 |
| PEG 1500 | 8 | 8 | 8 | 8 |
| POE(15)oleyl alcohol ether | 1 | 1 | 1 | 1 |
| Glucoside ascorbate | 2 | 2 | 2 | 2 |
| Potassium hydroxide | 0.2 | 0.2 | 0.2 | 0. 2 |
| Citric Acid | 0.01 | 0.01 | 0.01 | 0.01 |
| Sodium Citrate | 0.09 | 0.09 | 0.09 | 0.09 |
| Methylparaben | 0.2 | 0.2 | 0.2 | 0.2 |
| Example 1 | 0.5 | | | |
| Example 2 | | 0.5 | | |
| Comparative Example 1 | | | 0.5 | |
| Comparative Example 2 | | | | 0.5 |
| Ion-exchange water | to 100 | to 100 | to 100 | to 100 |
| Stability | ○ | ○ | × | ○ |
| Usability | ○ | ○ | × | Δ |

### Acidic Dye Material (Examples 7 and 8, Comparative Examples 7 and 8)

The acidic dye materials shown in Table 4 were produced by an ordinary method. The gel components and the results of evaluating the gel are shown. From the results of Table 4 it can be understood that the cosmetics of the invention have very excellent stability and usability.

**[Table 4]**

| | Example 7 | Example 8 | Comp. Ex. 7 | Comp. Ex. 8 |
|---|---|---|---|---|
| Acidic dye | 7 | 7 | 7 | 7 |
| Benzyl alcohol | 8 | 8 | 8 | 8 |
| Isopropyl alcohol | 1 | 1 | 1 | 1 |
| Citric acid | 2 | 2 | 2 | 2 |
| Example 1 | 0.5 | | | |
| Example 2 | | 0.5 | | |
| Comparative Example 1 | | | 0.5 | |
| Comparative Examples 2 | | | | 0.5 |
| Ion-exchange water | to 100 | to 100 | to 100 | to 100 |
| Stability | ○ | ○ | × | ○ |
| Usability | ○ | ○ | × | Δ |

### Oxidizing Dye Material (Examples 9 and 10, Comparative Examples 9 and 10)

A secondary agent (hydrogen peroxide solution) of each oxidizing dye material shown in Table 5 was prepared by an ordinary method. The gel components and the results of evaluating the gel are shown. It should be noted that the usability (the ease of handling and the dyeing ability) was evaluated based on the following criteria in a sensory test performed by a panel of nine specialists. From the results of Table 5 it can be understood that the cosmetics of the invention have excellent usability.

### <Ease of Handling>

ⓞ : All nine panelists responded that the cosmetic did not run and was applied to hair with ease.
○ : Six to eight panelists responded that the cosmetic did not run and was applied to hair with ease.
Δ : Three to five panelists responded that the cosmetic did not run and was applied to hair with ease.
× : Two or fewer panelists responded that the cosmetic did not run and was applied to hair with ease.

### <Dyeing Ability>

ⓞ : All nine panelists responded that the cosmetic had excellent dyeing ability.
○ : Six to eight panelists responded that the cosmetic had excellent dyeing ability.
Δ : Three to five panelists responded that the cosmetic had excellent dyeing ability.
× : Two or fewer panelists responded that the cosmetic had excellent dyeing ability.

**[Table 5]**

| | Example 9 | Example 10 | Comp. Ex. 9 | Comp. Ex. 10 |
|---|---|---|---|---|
| Hydrogen peroxide solution (30%) | 20 | 20 | 20 | 20 |
| Phosphoric ac i d | 0.2 | 0.2 | 0.2 | 0.2 |
| Disodium hydrogen phosphate | 0.2 | 0.2 | 0.2 | 0.2 |
| Sodium stannate | 0.02 | 0.02 | 0.02 | 0.02 |
| Methylparaben | 0.05 | 0.05 | 0.05 | 0.05 |
| Example 1 | 0.5 | | | |
| Example 2 | | 0.5 | | |
| Comparative Example 1 | | | 0.5 | |
| Comparative Example 2 | | | | 0.5 |
| Ion-exchange water | to 100 | to 100 | to 100 | to 100 |
| Stability | ○ | ○ | × | ○ |
| Usability (ease of handling) | ○ | ○ | × | Δ |
| Usability (dyeing ability) | ○ | ○ | Δ | ○ |

### Self-Tanning Gel (Examples 11 and 12, Comparative Examples 11 and 12)

The self-tanning gels shown in Table 6 were produced by an ordinary method. The gel components and the results of evaluating the gel are shown. Regarding the sensation during use, the sensory evaluation of the tactile sensation during use was performed by three specialized panelists. The evaluation rating was determined based on the following criteria.
4 Points: Very excellent.
3 Points: Excellent.
2 Points: Cannot say either way.
1 Point: Poor.
ⓞ : Average score between 3.5 or more and 4 or less.
○ : Average score of at least 3 but less than 3.5.
Δ : Average score of at least 2 but less than 3.
×: Average score less than 2.

**[Table 6]**

| | Example 11 | Example 12 | Comp. Ex. 11 | Comp. Ex. 12 |
|---|---|---|---|---|
| Ion-exchange water | Balance | Balance | Balance | Balance |
| Sodium pyrosulfite | 0.02 | 0.02 | 0.02 | 0.02 |
| EDTA 2-Na·2-hydrate | 0.03 | 0.03 | 0.03 | 0.03 |
| Dihydroxyacetone | 3 | 3 | 3 | 3 |
| 1,3-butylene glycol | 5 | 5 | 5 | 5 |
| Dynamite glycerin | 2 | 2 | 2 | 2 |
| Methylparaben | 0.17 | 0.17 | 0.17 | 0.17 |
| Phenoxyethanol | 0.3 | 0.3 | 0.3 | 0.3 |
| L-menthol | 0.005 | 0.02 | 0.02 | 0.02 |
| Ethanol | 5 | 5 | 5 | 5 |
| Caramel | 0.2 | 0.2 | 0.2 | 0.2 |
| Caffeine | 0.01 | 0.01 | 0.01 | 0.01 |
| Example 1 | 0.8 | | | |
| Example 2 | | 0.8 | | |
| Comparative Example 1 | | | 0.8 | |
| Comparative Example 2 | | | | 0.8 |
| Usage feel when applied | Ⓞ | Ⓞ | O | Δ |
| Stability of viscosity | Ⓞ | Ⓞ | × | O |
| Degree of dyeing | Ⓞ | Ⓞ | Δ | × |
| Overall evaluation | Ⓞ | Ⓞ | Δ | × |

The results of Table 6 show that the cosmetic of the invention has an excellent usage feel when applied, its viscosity stability is good, it has an excellent degree of dyeing, and its overall evaluation is superb.

### Self-Tanning Cream (Examples 13 and 14, Comparative Examples 13 and 14)

The self-tanning creams shown in Table 6 were prepared by an ordinary method. The gel components and the results of evaluating the gel are shown. Regarding the sensation during use, the sensory evaluation of the tactile sensation during use was performed by three specialized panelists. The evaluation rating was determined based on the following criteria.
4 Points: Very excellent.
3 Points: Excellent.
2 Points: Cannot say either way.
1 Point: Poor.
Ⓞ : Average score between 3.5 or more and 4 or less.
O : Average score of at least 3 but less than 3.5.
Δ: Average score of at least 2 but less than 3.
× : Average score less than 2.

**[Table 7]**

| | Example 13 | Example 14 | Comp. Ex. 13 | Comp. Ex. 14 |
|---|---|---|---|---|
| lon-exchange water | Balance | Balance | Balance | Balance |
| Sodium pyrosulfite | 0.02 | 0.02 | 0.02 | 0.02 |
| EDTA 2-Na·2-hydrate | 0.03 | 0.04 | 0.04 | 0.04 |
| Dihydroxyacetone | 5 | 5 | 5 | 5 |
| 1,3-butylene glycol | 5 | 7.2 | 7.2 | 7. 2 |
| Dynamite glycerin | 2 | 3 | 3 | 3 |
| Methylparaben | 0.17 | 0.24 | 0.24 | 0.24 |
| Phenoxyethanol | 0.3 | 0.3 | 0.3 | 0.3 |
| Example 1 | 0.8 | | | |
| Example 2 | | 0.8 | | |
| Comparative Example 1 | | | 0.8 | |
| Comparative Example 2 | | | | 0.8 |
| Polyoxyethylene polyoxypropylene cetyl ether | 0.5 | 0.5 | 0.5 | 0.5 |
| Decamethylcyclopentasiloxane | 5 | 5 | 5 | 5 |
| Usage feel when applied | Ⓞ | Ⓞ | O | × |
| Stability of viscosity | Ⓞ | Ⓞ | × | O |
| Degree of dyeing | Ⓞ | Ⓞ | Δ | × |
| Overall evaluation | Ⓞ | Ⓞ | Δ | × |

The results of Table 7 show that the cosmetic of the invention has an excellent usage feel when applied, its viscosity stability is good, it has an excellent degree of dyeing, and its overall evaluation is superb.

### INDUSTRIAL APPLICABILITY

The water-soluble thickener of the invention can stably thicken cosmetic preparations over a wide pH range without lowering the viscosity. When blended with cosmetic, the water-soluble thickener gives a very good usage feel, which could not be obtained with conventional thickeners. The water-soluble thickener of the invention therefore can be favorably adopted as a thickener for topical cosmetics and hair cosmetics, and thus it is possible to provide cosmetics that have an excellent feel when used and that have superb stability.

## Claims

1. A water-soluble thickener made of a copolymer that is obtained by copolymerizing 2-acrylamido-2-methylpropanesulfonic acid or its salt, hydroxyethylacrylamide, and a crosslinking monomer, or that is made of a copolymer that is obtained by further neutralizing said copolymer that has been obtained using an alkaline agent.

2. The water-soluble thickener according to claim 1, wherein the crosslinking monomer is N,N'-methylenebisacrylamide.

3. The water-soluble thickener according to claim 1 or 2, wherein a mole ratio of the 2-acrylamido-2-methylpropanesulfonic acid unit and the hydroxyethylacrylamide unit in the copolymer is 1:9 to 9:1.

4. A cosmetic in which the water-soluble thickener according to any one of claims 1 to 3 has been blended.

## Patentansprüche

1. Wasserlösliches Verdickungsmittel, hergestellt aus einem Copolymer, das erhalten wird durch Copolymerisieren von 2-Acrylamido-2-methylpropansulfonsäure oder deren Salz, Hydroxyethylacrylamid und eines Vernetzungsmonomeren, oder das hergestellt ist aus einem Copolymer, das erhalten wird durch weiterhin Neutralisieren des erhaltenen Copolymeren unter Verwendung eines alkalischen Mittels.

2. Wasserlösliches Verdickungsmittel nach Anspruch 1, wobei das Vernetzungsmonomer N,N'-Methylenbisacrylamid ist.

3. Wasserlösliches Verdickungsmittel nach Anspruch 1 oder 2, wobei das Molverhältnis der 2-Acrylamido-2-methylpropansulfonsäureeinheit und der Hydroxyethylacrylamideinheit in dem Copolymer 1:9 bis 9:1 beträgt.

4. Kosmetikum, in welches das wasserlösliche Verdickungsmittel gemäß mindestens einem der Ansprüche 1 bis 3 eingemischt worden ist.

## Revendications

1. Agent épaississant hydrosoluble, fait d'un copolymère obtenu par copolymérisation d'acide 2-acrylamido-2-méthyl-propane-sulfonique ou d'un sel de cet acide, d'hydroxyéthyl-acrylamide et d'un monomère agent de réticulation, ou fait d'un copolymère obtenu par neutralisation ultérieure, à l'aide d'un agent alcalin, dudit copolymère obtenu en premier lieu.

2. Agent épaississant hydrosoluble conforme à la revendication 1, pour lequel le monomère agent de réticulation est du N,N'-méthylène-bis(acrylamide).

3. Agent épaississant hydrosoluble conforme à la revendication 1 ou 2, dans lequel le rapport molaire des motifs dérivés de l'acide 2-acrylamido-2-méthyl-propane-sulfonique aux motifs dérivés de l'hydroxyéthyl-acrylamide dans le copolymère vaut de 1/9 à 9/1.

4. Produit cosmétique dans lequel un agent épaississant hydrosoluble conforme à l'une des revendications 1 à 3 a été incorporé.
